# EUROPEAN PATENT SPECIFICATION

(11) **EP 1 273 232 B1**
(45) Date of publication and mention of the grant of the patent: **21.06.2006**
(21) Application number: 02078910.3
(22) Date of filing: 07.08.1996
(51) Int. Cl.: A01N 43/653, C07D 249/12

(54) **Process for the preparation of the herbicide ethyl alpha-2-dichloro-5-¬4-(difluoromethyl)-4,5-dihydro-3-methyl-5-oxo-1h-1,2,4-triazol-1-yl|-4-fluorobenzenepropanoate**
Verfahren zur herstellung des herbizids alpha-2-dichloro-5-¬4-(difluoromethyl)-4,5-dihydro-3-methyl-5-oxo-1h-1,2,4-triazol-1-yl|-4-fluorophenylpropansäureethylester
Procédé de préparation de l'herbicide ethyle alpha-2-dichloro-5-¬4-(difluoromethyl)-4,5-dihydro-3-methyl-5-oxo-1h-1,2,4-triazol-1-yl|-4-fluorobenzenepropanoate

(30) Priority: 21.08.1995 US 2586 P
(43) Date of publication of application: 08.01.2003
(62) Divisional of application: 96926901.8
(73) Proprietor: FMC CORPORATION, Philadelphia, PA 19103 (US)
(72) Inventor: Ager, John W., Princeton, NJ 08540 (US); Polsz, Craig A., Newton, PA 18940 (US)
(74) Representative: W.P. Thompson & Co.

(56) References cited:
- US-A- 5 125 958

## Description

This invention relates to an improved process for carrying out the last step in the preparation of the herbicide ethyl α-2-dichloro-5-[4-(difluoromethyl)-4,5-dihydro-3-methyl-5-oxo-1H-1,2,4-triazol-1-yl]-4-fluorobenzenepropanoate, a Meerwein diazotization and arylation reaction

. The herbicide, which has the following structure, 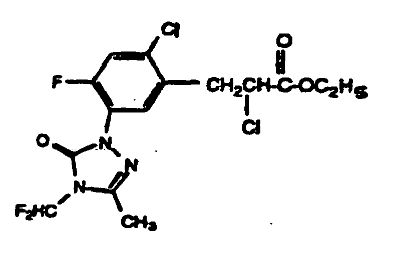 is disclosed and claimed in United States patent 5,125,958.

In common practice a Moorwein diazotization and arylation reaction is carried out in two steps. First, an arylamine is diazotized in an aqueous solution of, for example, sodium nitrite, and then the solution of diazotized amine is added to a solution of the compound to be arylated.

In U.S. 5,125,958 the last step in the preparation of the herbicide involved diazotization of the amine 4-difluoromethyl-4,5-dihydro-3-methyl-5-oxo-1-(5-amino-2-fluoro-4-chlorophenyl)-1H-1,2,4-triazole. However, since it had been found that when one attempted to diazotize this amine In aqueous solution, the 2-fluoro substituent was lost by hydrolysis to the corresponding phenol. Accordingly, the diazotization described in the patent was carried out in the conventional two steps, but in nonaqueous medium, with t-butyl nitrite as the source of the nitrite. The diazotized amine was then added to ethyl acrylate (the arylation) to yield the desired herbicide. While this procedure is satisfactory for laboratory production on a small scale, it is not satisfactory for large scale, commercial production. Not only is the supply of t-butyl nitrite too limited, but large scale production would require handling large quantities of the diazo compound, an undesirably hazardous operation.

Suprisingly, it has now been found that under the proper conditions the diazotization and arylation can be carried out simultaneously, with aqueous sodium nitrite, without loss of the 2-fluoro substituent and in surprisingly good yields.

The diazotization/arylation reaction sequence is as follows:

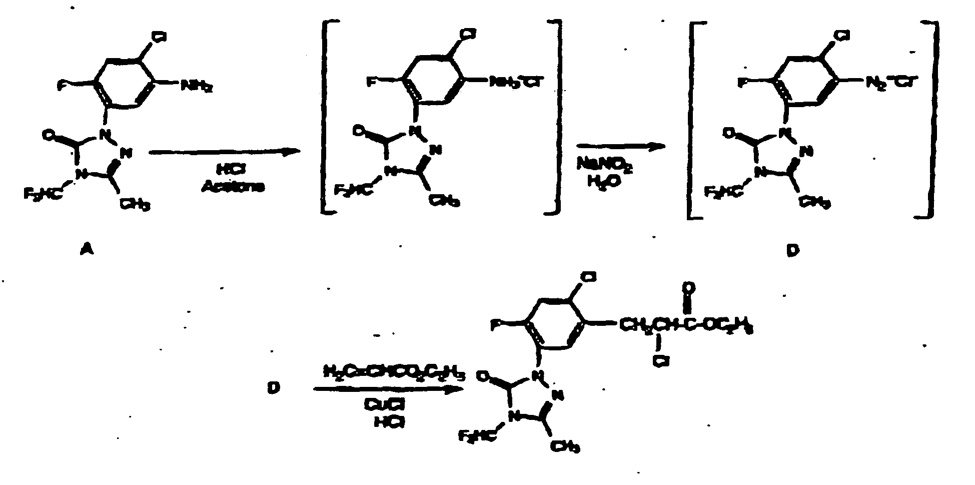

As noted above, in common practice the diazonium salt is first prepared and then added to the compound to be arylated. In the process of the invention the hydrochloride salt of the amine A is diazotized with aqueous sodium nitrite in the presence of ethyl acrylate, the material to be arylated. The diazonium chloride (D) produced by the diazotization of the amine hydrochloride with sodium nitrite reacts with ethyl acrylate in the presence of a catalytic amount of a Meerwein arylation catalyst, e.g. cuprous or cupric chloride. The reaction is preferably carried out at low temperature in acetone. The use of acetone as the solvent allows the use of sodium nitrite in a minimum of water. Acetone also facilitates the recycling of the copper(I) chloride catalyst. Another advatage of the process is that the fact that the diazotization/arylation reactions are occurring simultaneously minimizes the probability of the diazotization product reacting with itself.

The process starts with the preparation of a solution of the amine A in acetone, preferably in an inert atmosphere, e.g. nitrogen. A useful ratio of acetone to amine is in the range of 10 to 30 equivalents of acetone to one equivalent of amine, preferably 20 to 25 equivalents of acetone to one of amine. The solution may be filtered to remove any insoluble material that may be present. To the amine solution is added, with stirring, a Meerwein arylation catalyst, for example, copper(I) chloride or copper(II) chloride, preferably copper(I) chloride, and, again, an inert atmosphere is preferred. (Stirring is maintained throughout the course of the reaction.) The amount of copper(I) chloride is important in the process of the present invention. If too little is used, the reaction is slower, and the yield of the final product is lower. Too much copper(I) chloride in the reaction mixture results in dechlorination of the final product. Therefore, a useful ratio of copper(I) chloride to amine is in the range of 0.05 to 1.0 equivalent of copper(I) chloride to one equivalent of amine, preferably 0.1 to 0.15 equivalent to one. The reaction mixture is then cooled to below 0 °C, preferably to about -10°C, and the amine salt is prepared by addition of concentrated hydrochloric acid, or anhydrous hydrogen chloride, preferably anhydrous hydrogen chloride added below the surface of the reaction mixture. During the addition the reaction mixture is maintained at a temperature of -20 °C to 30 °C, preferably -10 °C to 10 °C. The preparation of the amine salt requires about 30 to 120 minutes, preferably about 45 to 90 minutes. The amount of ethyl acrylate that is next added to the amine salt slurry is important. A large excess of ethyl acrylate is required to obtain optimum yields from this process. If the excess is reduced, the yield of final product is reduced. A useful ratio of ethyl acrylate to amine has been found to be 5 to 20 equivalents of ethyl acrylate to one equivalent of amine, preferably 10 to 15 equivalents to one. The time needed to add the ethyl acrylate is relatively unimportant. However, the reaction mixture temperature is maintained below 10 °C throughout the addition and is controlled to some degree by the addition of the ethyl acrylate. The reaction mixture is then brought to about 0 °C, and an aqueous solution of sodium nitrite is added to the reaction mixture. This step is a critical step in the process of the present invention. The diazotization/arylation reactions are occurring simultaneously. During the diazotization step water must be kept to a minimum. A large amount of water results in hydrolysis of the diazo intermediate, yielding a phenol by-product. To minimize the production of by-products a useful ratio of sodium nitrite to amine is in the range of 1.0 to 2.0 equivalents of sodium nitrite to one equivalent of amine, preferably 1.4 to 1.7 equivalents to one. To keep the amount of water to a minimum, it is advantageous to use a concentrated aqueous solution of sodium nitrite for the diazotization step. A saturated aqueous solution of sodium nitrite contains about 40% (wt/wt) of sodium nitrite. A useful concentration of the aqueous solution of sodium nitrite is, therefore, about 20 % to 40 % (wt/wt) sodium nitrite, preferably 35 % to 40% sodium nitrite. Reaction mixture temperatures are also important in optimizing the diazotization/arylation reaction. Temperatures above 10°C increase the probability of by-product formation. A useful range of reaction mixture temperatures for the optimization of the process step is -10 °C to 20 °C, preferably 0 °C to 10°C. The rate of addition of the aqueous sodium nitrite solution is also important in optimizing yield. Fast rates of addition in the process of the present invention result in lower yields of product. The optimum rate of addition requires about 1 to 6 hours, preferably 2 to 3 hours. The addition of the aqueous sodium nitrite solution is done below the surface of the stirred reaction mixture. The reaction mixture is then stirred for a period of 15 to 90 minutes, preferably 20 to 40 minutes, to allow completion of the reaction. At this point crude yields of product in the range of 80-88% are obtained. The purity of the product may be improved by washing, followed by distillation. During the washing step the temperature is maintained at about -10 °C to 20 °C, preferably 5 °C to 10 °C, and in the preferred washing sequence the reaction mixture is washed first with a dilute aqueous acid, for example, 5% hydrochloric acid, then with a dilute aqueous base, for example 5% sodium hydroxide, and finally with a sodium chloride solution. Upon completion of the wash steps, the volatile material in the reaction mixture, i. e., acetone/ethyl acrylate, may be removed at a pot temperature of 30 °C to 80 °C, preferably 40 °C to 65 °C, under a reduced pressure of 2700 to 9300 Pa (20 mm to 70 mmHg), preferably 6000 to 7300 Pa (45 mm to 55 mm Hg). The removal of volatile material is considered complete when the ethyl acrylate concentration is below about five percent. Ethyl acrylate is known to polymerize under certain conditions. As a precautionary measure, an antioxidant and a free-radical inhibitor are added to the distillation overhead system. There is, however, no evidence that polymerization occurs in the reaction vessel during the reaction, wash steps, or during removal of the volatile material**.**

For further purification of the product the non-volatile material containing the reaction product may be distilled in a short path evaporaror or wiped-film still. In order to increase the efficiency of the distillation process, the reaction product may be passed (degassing step) through the still at an evaporator temperature of 100°C to 140°C and a pressure of 1300 to 2700 Pa (10 mm to 20 mmHg), preferably 120°C to 130°C and a pressure of 1700 to 2400 Pa (13 mm to 18 mm Hg), to remove any remaining ethyl acrylate or other low-boiling materials. The degassed reaction product may then be passed once through the still at an evaporator temperature of 160 °C to 180°C and a pressure of 130 to 670 Pa (1.0 mm to 5.0 mm Hg), preferably 170°C to 180°C and a pressure of 130 to 270 Pa (1.0 mm to 2.0 mm Hg). The non-volatile material from the first pass through the still may then be passed a second time through the still at an evaporator temperature of 160°C to 200°C and a pressure of 4 to 130 Pa (0.03 mm to 1.0 mm Hg), preferably 160°C to 180°C and a pressure of 4 to 70 Pa (0.03 mm to 0.5 mm Hg). The rate of feed through the still will be governed by the size of the still. It is understood that the conditions for degassing and distillation will vary, depending on the apparatus used. Operation under preferred conditions has given a typical percent yield of distilled ethyl α-2-dichloro-5-[4-(difluoromethyl)-4,5-dihydro-3-methyl-5-oxo-1H-1,2,4-triazol-1-yl]-4-fluorobenzenepropanoate of 75-80% (based on the amount of starting amine); at a purity of about 91% (as determined by high pressure liquid chromatography).

### EXAMPLE 1

### PREPARATION OF ETHYL α-2-DICHLORO-5-[4-(DIFLUOROMETHYL)-4,5-DIHYDRO-3-METHYL-5-OXO-1H-1,2,4-TRIAZOL-1-YL]-4-FLUORO-BENZENEPROPANOATE, LABORATORY SCALE

A two-liter, jacketed resin flask was fitted with a thermometer, nitrogen inlet tube, outlet tube connected to a carbon trap (to minimize the escape of the odor of ethyl acrylate), and a fitting consisting of an eight-inch, large bore, teflon-coated hypodermic needle attached to a 50 mL syringe that was in turn connected to a syringe pump. In a separate vessel, 140.4 grams (0.46 mole-1.0 equiv.) of 4-difluoromethyl-4,5-dihydro-3-methyl-5-oxo-1-(5-amino-2-fluoro-4-chlorophenyl)-1H-1,2,4-triazole was dissolved in 600 grams (10.33 moles-22.5 equiv.) of acetone. The solution was cooled to 0 °C, and filtered into the resin flask. With stirring, 56.2 grams (1.54 moles-3.4 equiv.) of anhydrous hydrogen chloride gas was then bubbled into the triazole/acetone solution during a 15-20 minute period. Upon completion of the addition 6.1 grams (0.06 mole-0.13 equiv.) of copper(I) chloride was added. With continued stirring the reaction mixture was cooled to 0 °C under a nitrogen atmosphere, and 640.4 grams (6.33 moles-13.8 equiv.) of ethyl acrylate was added. The reaction mixture was again cooled to 0 °C, and a solution of 48.1 grams (0.69 mole-1.5 equiv.) of sodium nitrite dissolved in 88.1 grams (4.9 moles-10.7 equiv.) of water was added. The nitrite/water solution was added to the reaction mixture at a rate of about 0.48 mL/minute from the syringe, with the needle outlet below the surface of the reaction mixture.

The complete addition required about 3.75 hours, after which time the reaction mixture was stirred for an additional 45 minutes at 0 °C. The reaction was then quenched with 200 mL of water, after which the reaction mixture was stirred for about 15 minutes. The organic layer was separated and washed with one portion each of 200 mL of aqueous 1 N hydrochloric acid, 200 mL of water, and a 1:1 mixture of aqueous 5% sodium hydroxide and ethyl acetate wherein the volume of sodium hydroxide solution and ethyl acetate were each equal to the volume of the organic layer. The organic layer was then concentrated at 70 °C under vacuum, yielding 193.3 grams (assay-84.6%, crude yield-86.4%) of ethyl a-2-dichloro-5-[4a(difluoromethyl)-4,5-dihydro-3-methyl-oxo-1H-1,2,4-triazol-1-yl]-4-fluorobenzenepropanoate. The concentrated product was passed through a 0.051 m (two-inch) 0.023m² (0.25ft²) Pope Wiped-Film Still at 160°C at 104 Pa (0.8 mm Hg) to remove lower boiling impurities. The non-volatile material was passed a second time through the wiped-film still at 220 °C 130 Pa (1 mm Hg) to distill the product from high-boiling impurities, yielding 134.9 grams (assay-92 %, distilled yield-65.6%) of ethyl α-2-dichioro-5-[4-(difluoromethyl)-4,5-dihydro-3-methyl-5-oxo-1H-1,2,4-triazol-1-yl]-4-fluorobenzenepropanoate.

### EXAMPLE 2

### PREPARATION OF ETHYL α-2-DICHLORO-5-[4-(DIFLUOROMETHYL)-4,5-DIHYDRO-3-METHYL-5-OXO-1H-1,2,4-TRIAZOL-1-YL]-4-FLUOROBENZENEPROPANOATE, PILOT PLANT SCALE

Several batches of product were prepared according to the following procedure. Acetone, 207.5 kg (457.4 pounds) 3.58 kg-moles (7.89 Ibs-moles, 22 equiv.) was charged by pressure differential into a 760L (200 gallon) jacketed reactor, followed by 48.5 kg (107 pounds) 0.16 kg-mole (0.36 1b-mole, 1.0 equiv.) of 98% 4-difluoromethyl-4,5-dihydro-3-methyl-5-oxo-1-(5-amino-2-fluoro-4-chlorophenyl)-1H-1,2,4-triazole. The mixture was stirred and optionally filtered at ambient temperature to remove solid impurities. The solution was recharged into the 760 L (200 gallon) reactor, stirred, and purged with nitrogen. To this was then added 2.09 kg (4.6 pounds) 0.02 kg-mole (0.05 lb-mole, 0.13 equiv.) of copper(I) chloride. Upon completion of the addition the mixture was cooled to about -10 °C by circulating a cold brine solution through the jacket of the reactor. The reactor was closed, and the nitrogen purge was stopped. Anhydrous hydrogen chloride 14.83 kg (32.7 pounds) 0.4 kg mole (0.9 lb-mole, 2.5 equiv.), was then bubbled in below the surface of the reaction mixture at a rate of about 0.25 kg/min (0.55 lb/min.), while the temperature of the reaction mixture was maintained between -10 °C and 10 °C. The complete addition required about one hour. Upon completion of addition, the reactor was opened to atmospheric pressure. The reaction mixture temperature was then brought to about 0 °C, and 219.54 kg (484 pounds) 2.20 kg-moles (4.84 l1b-moles, 13.5 equiv.) of ethyl acrylate was added by gravity. Upon completion of the addition a solution of 16.83 kg (37.1 pounds) 2.20 kg-moles (4.84 lb-moles, 1.5 equiv) of sodium nitrite in 31.30 kg (69 pounds) 1.74 kg-moles (3.83 lb-moles, 10.6 equiv.) of water was added at a rate of about 0.27 kg (0.6 pound) minute, below the surface of the reaction mixture. The reaction mixture temperature was maintained between 0 °C and 5 °C. The complete addition required about three hours, after which time the reaction mixture was stirred for 30 minutes. While the reaction mixture temperature was kept between 5°C and 10°C, the reaction mixture was washed in turn with 66.68 kg (147 pounds) of water, 66.59 kg (146.8 pounds) of aqueous 5% hydrochloric acid 69.4 kg (153 pounds) of aqueous 5% sodium hydroxide, and 68.04 kg (150 pounds) of water. The ethyl acrylate and acetone were then removed from the reaction mixture by distillation at about 65°C/6666 Pa (65°C/50 mm Hg). To prevent polymerization the distilled ethyl acrylate and acetone mixture was stabitized by a solution consisting of 0.82 kg (0.18 pound) of 4-methoxyphenol, 0.82 kg (0.18 pound) of phenothiazine, and 25.4 kg (56 pounds) of toluene, which was circulated through the condenser and distillate receiver. The temperature of the residue-product was maintained at about 45°C as it was drummed for future use. The yield of crude product, ethyl α-2-dichloro-5-[4-(difluoromethyl)-4,5-dihydro-3-methyl-5-oxo-1H-1,2,4-triazol-1-yl]-4-flourobenzenepropanoate, was 69.0 kg (152.2 pounds). A number of batches prepared as described above were combined. The combination was passed through a wiped-film distillation still, first under low vacuum to remove low-boiling material (degassed), then twice under high vacuum to obtain distilled product. The following conditions were used with the wiped-film still:

| | Degassing | | |
|---|---|---|---|
| | Step | First Pass | Second Pass |
| Feed Temp (°C) | 60 | 65 | 65 |
| Evaporator Temperature (°C) | 125 | 175 | 193 |
| Condenser Coolant (°C) | -2 | 70 | 60 |
| Agitator speed (rpm) | 425 | 425 | 425 |
| Vacuum Pa (mm Hg) | 2000(15) | 80(0.6) | 66.7(0.5) |

The percent yield of distilled ethyl α-2-dichloro-5-[4-(difluoromethyl)-4,5-dihydro-3-methyl-5-oxo-1H-1,2,4-triazol-1-yl]-4-fluorobenzenepropanoate was 65% (based on the amount of starting amine); purity was 91 % (as determined by means of high pressure liquid chromatography).

### EXAMPLE 3

### PREPARATION OF ETHYL α-2-DICHLORO-5-[4-(DIFLUOROMETHYL)-4,5-DIHYDRO-3-METHYL-5-OXO-1H-1,2,4-TRIAZOL-1-YL]-4-FLUORO-BENZENEPROPANOATE, PILOT PLANT SCALE

Several batches of product were prepared according to the following procedure. A 3785 L (1000 gallon) jacketed reactor was purged with nitrogen, and 231 kg (510.0 pounds) 0.76 kg-moles (1.71 lb-moles, 1.0 equiv.) of 98% 4-difluoromethyl-4,5-dihydro-3-methyl-5-oxo-1-(5-amino-2-fluoro-4-chlorophenyl)-1H-1,2,4-triazole was charged into the reactor. To this was then charged 9.98 kg (22.0 pounds) 0.10 kg-moles (0.22 lb-mole, 0.13 equiv.) of copper(l) chloride. The reactor was sealed and again purged with nitrogen. Acetone, 988.3 kg (2178.8 pounds) 17.04 kg-moles (37.57 lb-moles, 22.0 equiv.) was then charged into the reactor by means of a positive displacement pump. Upon completion of the addition the mixture was stirred and cooled to between -10 °C and 0 °C by circulating a cold brine solution through the jacket of the reactor. Anhydrous hydrogen chloride 70.67 kg (155.8 pounds) 1.94 kg-mole (4.27 lb-mole, 2.5 equiv.), was then bubbled in below the surface of the reaction mixture at a rate of about 1.13 kg/min. (2.5 lb/min.), while the reaction mixture temperature was held below 10 °C. The anhydrous hydrogen chloride addition time was typically completed in one to two hours. Upon completion of the hydrogen chloride feed the reactor was vented to atmospheric pressure. The reaction mixture temperature was then brought to about 0 °C, and 1045.6 kg (2305.2 pounds) 10.48 kg-moles (23.1 lb-moles, 13.5 equiv.) of ethyl acrylate was charged into the reactor by means of a positive displacement pump. Upon completion of the addition 200.4 kg (441.8 pounds) of an aqueous 40 weight percent 1.16 kg-mole (2.56 lb-mole, 1.5 equiv.) sodium nitrite solution was charged below the surface of the reaction mixture at a rate of about 0.82 kg/min. (1.8 lbs/minute). The reaction mixture temperature was maintained between 0 °C and 5 °C. The complete addition required about four hours, after which time the reaction mixture was stirred for 30 minutes. The reaction was considered complete when the amount of unreacted amine (solvent free) remaining in the reaction mixture was less than about 0.5 area percent by gas chromatography. Upon completion of the reaction the reaction mixture was maintained at 5 °C to 10 °C and washed with two portions of 317.5 kg (700 pounds) each of an aqueous 5% hydrochloric acid solution, two portions of 330.7 kg (729 pounds) each of an aqueous 5% sodium hydroxide solution, and one portion of 324.3 kg (715 pounds) of an aqueous 10% sodium chloride solution. The ethyl acrylate and acetone were then removed from the reaction mixture by distillation at between 65 °C and 75°C at less than 1333.2 Pa (10 mm Hg), until the concentration of ethyl acrylate in the solution was less than about five percent. To prevent polymerization, the ethyl acrylate and acetone mixture distillate was stabilized by a solution consisting of 0.095 kg (0.21 pound) of 4-methoxyphenol, 0.095 kg (0.21 pound) of phenothiazine, and 12.71 kg (28 pounds) of toluene, which was circulated through the condenser and distillate receiver. The temperature of the residue-product was maintained at about 45 °C as it was drummed for future use. The yield of crude product, ethyl α-2-dichloro-5-[4-(difluoromethyl)-4,5-dihydro-3-methyl-5-oxo-1H-1,2,4-triazol-1-yl]-4-benzenepropanoate, from this reaction at this point was typically about 82%. A number of batches prepared as described above were combined. The combination was passed through a short path evaporator (SPE), first under low vacuum to remove low-boiling material (degassing), then twice under high vacuum to obtain distilled product. The following conditions were used with the SPE:

| | Degassing | | |
|---|---|---|---|
| | Step | First Pass | Second Pass |
| Feed Temp (°C) | 65 | 65 | 65 |
| Evaporator Tem-perature (°C) | 100 | 170 | 170 |
| Condenser Coolant (°C) | -30 | 70 | 70 |
| Agitator speed* (rpm) | 200 | 200 | 200 |
| Vacuum Pa (mm Hg) | 1333(10) | 385(2.8) | 37.3(0.28) |

| | | | |
|---|---|---|---|
| *The agitator speed will vary depending on the size of the SPE; these figures are for an 0.743 m² (8ft²) SPE. | | | |

The percent yield of ethyl α-2-dichloro-5-[4-(difluoromethyl)-4,5-dihydro-3-methyl-5-oxo-1H-1,2,4-triazol-1-yl]-4-fluorobenzenepropanoate distilled was 80% (based on the amount of starting amine); purity was 91% (as determined by high pressure liquid chromatography).

## Claims

1. A process for the preparation of a compound of formula (I): 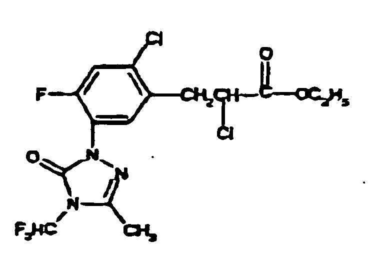 comprising: (i) forming a diazotized salt of 4-difluoro-methyl-4,5-dihydro-3-methyl-5-oxo-1-(5-amino-2-fluoro-4-chlorophenyl)-1H-1,2,4-triazole by reacting a salt of 4-difluoro-methyl-4,5-dihydro-3-methyl-5-oxo-1-(5-amino-2-fluoro-4-chlorophenyl)-1H-1,2,4-triazole with aqueous sodium nitrite; and (ii) simultaneously reacting said diazotized salt of 4-difluoro-methyl-4,5-dihydro-3-methyl-5-oxo-1-(5-amino-2-fluoro-4-chlorophenyl)-1H-1,2,4-triazole, as it is produced in step (i), with ethyl acrylate in the presence of a Meerwein arylation catalyst to obtain a compound of formula (I).

2. A process as claimed in claim 1, wherein said Meerwein arylation catalyst is selected from the group consisting of cuprous chloride and cupric chloride.

3. A process as claimed in claim 1 or 2, wherein the simultaneous reaction in step (ii) further occurs in the presence of hydrochloric acid or anhydrous hydrogen chloride.

4. A process as claimed in any one of claims 1 to 3, wherein said salt of 4-difluoro-methyl-4,5-dihydro-3-methyl-5-oxo-1-(5-amino-2-fluoro-4-chlorophenyl)-1H-1,2,4-triazole is a hydrochloride salt.

5. A process for the preparation of a compound of formula (I): 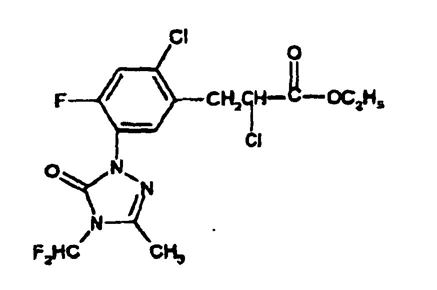 comprising the step of adding aqueous sodium nitrite to a solution comprising a salt of 4-difluoro-methyl-4,5-dihydro-3-methyl-5-oxo-1-(5-amino-2-fluoro-4-chlorophenyl)-1H-1,2,4-triazole, ethyl acrylate and a Meerwein arylation catalyst to form formula (I).

6. A process as claimed in claim 5, wherein said Meerwein arylation catalyst is selected from the group consisting of cuprous chloride and cupric chloride.

7. A process as claimed in claim 6, wherein said solution contains acetone.

8. A process as claimed in claim 5, 6 or 7, wherein said ethyl acrylate is added in 5 to 20 equivalents to one equivalent of said salt of 4-difluoro-methyl-4,5-dihydro-3-methyl-5-oxo-1-(5-amino-2-fluoro-4-chlorophenyl)-1H-1,2,4-triazole.

9. A process as claimed in any one of claims 5 to 8, wherein said aqueous sodium nitrite is added in 1.0 to 2.0 equivalents of said salt of 4-difluoro-methyl-4,5-dihydro-3-methyl-5-oxo-1-(5-amino-2-fluoro-4-chlorophenyl)-1H-1,2,4-triazole.

10. A process as claimed in any one of claims 5 to 9, wherein said ethyl acrylate is added in 5 to 20 equivalents to one equivalent of said salt of 4-difluoro-methyl-4,5-dihydro-3-methyl-5-oxo-1-(5-amino-2-fluoro-4-chlorophenyl)-1H-1,2,4-triazole, and said aqueous sodium nitrite is added in 1.0 to 2.0 equivalents of said salt of 4-difluoro-methyl-4,5-dihydro-3-methyl-5-oxo-1-(5-amino-2-fluoro-4-chlorophenyl)-1H-1,2,4-triazole.

11. A process as claimed in claim 10, wherein said ethyl acrylate is added in 5 to 20 equivalents to one equivalent of said salt of 4-difluoro-methyl-4,5-dihydro-3-methyl-5-oxo-1-(5-amino-2-fluoro-4-chlorophenyl)-1H-1,2,4-triazole, and said aqueous sodium nitrite is added in 1.0 to 2.0 equivalents of said salt of 4-difluoromethyl-4,5-dihydro-3-methyl-5-oxo-1-(5-amino-2-fluoro-4-chlorophenyl)-1 H-1,2,4-triazole.

12. A process as claimed in claim 11, wherein said ethyl acrylate and queous sodium nitrite are added at a temperature below 10°C.

13. A process as claimed in claim 5, wherein said solution further contains hydrochloric acid or anhydrous hydrogen chloride.

## Patentansprüche

1. Verfahren zur Herstellung einer Verbindung der Formel (I): 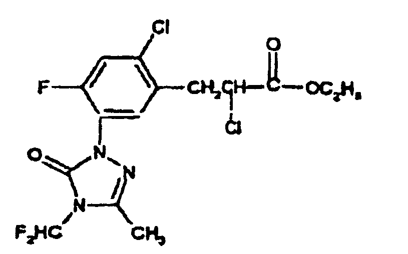 umfassend: (i) Bildung eines diazotierten Salzes von 4-Difluormethyl-4,5-dihydro-3-methyl-5-oxo-1-(5-amino-2-fluor-4-chlorphenyl)-1H-1,2,4-triazol durch Reaktion eines Salzes von 4-Difluormethyl-4,5-dihydro-3-methyl-5-oxo-1-(5-amino-2-fluor-4-chlorphenyl)-1H-1,2,4-triazol mit wässrigem Natriumnitrit; und (ii) simultane Reaktion von genanntem diazotierten Salz von 4-Difluormethyl-4,5-dihydro-3-methyl-5-oxo-1-(5-amino-2-fluor-4-chlorphenyl)-1H-1,2,4-triazol, wie es in Schritt (i) hergestellt ist, mit Ethylacrylat in Gegenwart eines Katalysators zur Meerwein-Arylierung, um eine Verbindung der Formel (I) zu erhalten.

2. Verfahren nach Anspruch 1, worin genannter Katalysator zur Meerwein-Arylierung aus der Gruppe ausgewählt ist, die aus Kupfer(I)-chlorid und Kupfer(II)-chlorid besteht.

3. Verfahren nach Anspruch 1 oder 2, worin simultane Reaktion in Schritt (ii) weiter in Gegenwart von Chlorwasserstoffsäure oder wasserfreiem Chlorwasserstoff erfolgt.

4. Verfahren nach einem der Ansprüche 1 bis 3, worin genanntes Salz von 4-Difluormethyl-4,5-dihydro-3-methyl-5-oxo-1-(5-amino-2-fluor-4-chlorphenyl)-1 H-1,2,4-triazol ein Hydrochloridsalz ist.

5. Verfahren zur Herstellung einer Verbindung der Formel (I): 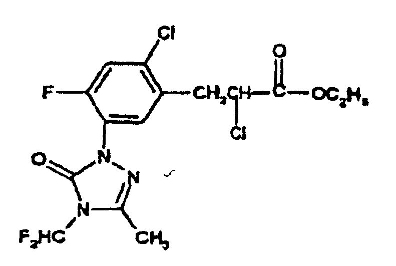 umfassend den Schritt der Zugabe von wässrigem Natriumnitrit zu einer Lösung, die ein Salz von 4-Difluormethyl-4,5-dihydro-3-methyl-5-oxo-1-(5-amino-2-fluor-4-chlorphenyl)-1H-1,2,4-triazol, Ethylacrylat und einen Katalysator zur Meerwein-Arylierung umfasst, zur Bildung der Formel (I).

6. Verfahren nach Anspruch 5, worin genannter Katalysator zur Meerwein-Arylierung aus der Gruppe ausgewählt ist, die aus Kupfer(I)-chlorid und Kupfer(II)-chlorid besteht.

7. Verfahren nach Anspruch 6, worin genannte Lösung Aceton enthält.

8. Verfahren nach Anspruch 5, 6 oder 7, worin genanntes Ethylacrylat in 5 bis 20 Äquivalenten zu einem Äquivalent von genanntem Salz von 4-Difluormethyl-4,5-dihydro-3-methyl-5-oxo-1-(5-amino-2-fluor-4-chlorphenyl)-1H-1,2,4-triazol zugegeben wird.

9. Verfahren nach einem der Ansprüche 5 bis 8, worin genanntes wässriges Natriumnitrit in 1,0 bis 2,0 Äquivalenten von genanntem Salz von 4-Difluormethyl-4,5-dihydro-3-methyl-5-oxo-1-(5-amino-2-fluor-4-chlorphenyl)-1H-1,2,4-triazol zugegeben wird.

10. Verfahren nach einem der Ansprüche 5 bis 9, worin genanntes Ethylacrylat in 5 bis 20 Äquivalenten zu einem Äquivalent von genanntem Salz von 4-Difluormethyl-4,5-dihydro-3-methyl-5-oxo-1-(5-amino-2-fluor-4-chlorphenyl)-1H-1,2,4-triazol zugegeben wird, und genanntes wässriges Natriumnitrit in 1,0 bis 2,0 Äquivalenten von genanntem Salz von 4-Difluormethyl-4,5-dihydro-3-methyl-5-oxo-1-(5-amino-2-fluor-4-chlorphenyl)-1H-1,2,4-triazol zugegeben wird.

11. Verfahren nach Anspruch 10, worin genanntes Ethylacrylat in 5 bis 20 Äquivalenten zu einem Äquivalent von genanntem Salz von 4-Difluormethyl-4,5-dihydro-3-methyl-5-oxo-1-(5-amino-2-fluor-4-chlorphenyl)-1H-1,2,4-triazol zugegeben wird, und genanntes wässriges Natriumnitrit in 1,0 bis 2,0 Äquivalenten von genanntem Salz von 4-Difluormethyl-4,5-dihydro-3-methyl-5-oxo-1-(5-amino-2-fluor-4-chlorphenyl)-1H-1,2,4-triazol zugegeben wird.

12. Verfahren nach Anspruch 11, worin genanntes Ethylacrylat und wässriges Natriumnitrit bei einer Temperatur unter 10°C zugegeben werden.

13. Verfahren nach Anspruch 5, worin genannte Lösung weiter Chlorwasserstoffsäure oder wasserfreien Chlorwasserstoff enthält.

## Revendications

1. Procédé de préparation d'un composé de formule (I) : 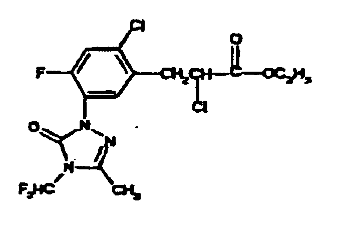 comprenant : (i) la formation d'un sel diazoté de 4-difluorométhyl-4,5-dihydro-3-méthyl-5-oxo-1-(5-amino-2-fluoro-4-chlorophényl)-1H-1,2,4-triazole en faisant réagir un sel de 4-difluorométhyl-4,5-dihydro-3-méthyl-5-oxo-1-(5-amino-2-fluoro-4-chlorophényl)-1H-1,2,4-triazole avec du nitrite de sodium aqueux ; et (ii) la mise en réaction simultanée dudit sel diazoté de 4-difluorométhyl-4,5-dihydro-3-méthyl-5-oxo-1-(5-amino-2-fluoro-4-chlorophényl)-1H-1,2,4-triazole, tel qu'il est produit dans l'étape (i), avec de l'acrylate d'éthyle en présence d'un catalyseur d'arylation de Meerwein afin d'obtenir un composé de formule (I).

2. Procédé selon la revendication 1, dans lequel ledit catalyseur d'arylation de Meerwein est choisi parmi le groupe constitué de chlorure cuivreux et de chlorure cuivrique.

3. Procédé selon la revendication 1 ou 2, dans lequel la réaction simultanée dans l'étape (ii) se produit en outre en présence d'acide chlorhydrique ou de chlorure d'hydrogène anhydre.

4. Procédé selon l'une quelconque des revendications 1 à 3, dans lequel ledit sel de 4-difluorométhyl-4,5-dihydro-3-méthyl-5-oxo-1-(5-amino-2-fluoro-4-chlorophényl)-1H-1,2,4-triazole est un sel de chlorhydrate.

5. Procédé de préparation d'un composé de formule (I) : 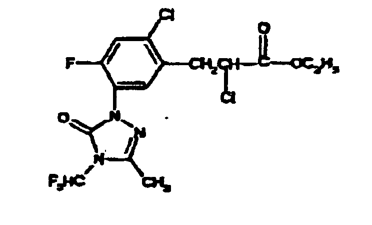 comprenant l'étape consistant à ajouter du nitrite de sodium aqueux à une solution comprenant un sel de 4-difluorométhyl-4,5-dihydro-3-méthyl-5-oxo-1-(5-amino-2-fluoro-4-chlorophényl)-1H-1,2,4-triazole, de l'acrylate d'éthyle et un catalyseur d'arylation de Meerwein afin de former la formule (I).

6. Procédé selon la revendication 5, dans lequel ledit catalyseur d'arylation de Meerwein est choisi parmi le groupe constitué de chlorure cuivreux et de chlorure cuivrique.

7. Procédé selon la revendication 6, dans lequel ladite solution contient de l'acétone.

8. Procédé selon la revendication 5, 6 ou 7, dans lequel ledit acrylate d'éthyle est ajouté en une quantité de 5 à 20 équivalents pour un équivalent dudit sel de 4-difluorométhyl-4,5-dihydro-3-méthyl-5-oxo-1-(5-amino-2-fluoro-4-chlorophényl)-1H-1,2,4-triazole.

9. Procédé selon l'une quelconque des revendications 5 à 8, dans lequel ledit nitrite de sodium aqueux est ajouté en une quantité de 1,0 à 2,0 équivalents dudit sel de 4-difluorométhyl-4,5-dihydro-3-méthyl-5-oxo-1-(5-amino-2-fluoro-4-chlorophényl)-1H-1,2,4-triazole.

10. Procédé selon l'une quelconque des revendications 5 à 9, dans lequel ledit acrylate d'éthyle est ajouté en une quantité de 5 à 20 équivalents pour un équivalent dudit sel de 4-difluorométhyl-4,5-dihydro-3-méthyl-5-oxo-1-(5-amino-2-fluoro-4-chlorophényl)-1H-1,2,4-triazole, et ledit nitrite de sodium aqueux est ajouté en une quantité de 1,0 à 2,0 équivalents dudit sel de 4-difluorométhyl-4,5-dihydro-3-méthyl-5-oxo-1-(5-amino-2-fluoro-4-chlorophényl)-1 H-1,2,4-triazole.

11. Procédé selon la revendication 10, dans lequel ledit acrylate d'éthyle est ajouté en une quantité de 5 à 20 équivalents pour un équivalent dudit sel de 4-difluorométhyl-4,5-dihydro-3-méthyl-5-oxo-1-(5-amino-2-fluoro-4-chlorophényl)-1H-1,2,4-triazole, et ledit nitrite de sodium aqueux est ajouté en une quantité de 1,0 à 2,0 équivalents dudit sel de 4-difluorométhyl-4,5-dihydro-3-méthyl-5-oxo-l-(5-amino-2-fluoro-4-chlorophényl)-1H-1,2,4-triazole.

12. Procédé selon la revendication 11, dans lequel lesdits acrylate d'éthyle et nitrite de sodium aqueux sont ajoutés à une température inférieure à 10°C.

13. Procédé selon la revendication 5, dans lequel ladite solution contient en outre de l'acide chlorhydrique ou du chlorure d'hydrogène anhydre.
